# EUROPÄISCHE PATENTSCHRIFT

(11) **EP 3 384 848 B1**
(45) Veröffentlichungstag und Bekanntmachung des Hinweises auf die Patenterteilung: **23.06.2021**
(21) Anmeldenummer: 17164760.5
(22) Anmeldetag: 04.04.2017
(51) Int. Cl.: A61B 6/03, A61B 6/00, G06T 7/00

(54) **FLEXIBLER EINSATZ EINES KLINISCHEN ENTSCHEIDUNGSUNTERSTÜTZUNGSSYSTEMS**
FLEXIBLE FITTING OF A CLINICAL DECISION-MAKING SUPPORT SYSTEM
UTILISATION POLYVALENTE D'UN SYSTÈME D'AIDE À LA DÉCISION CLINIQUE

(43) Veröffentlichungstag der Anmeldung: 10.10.2018
(73) Patentinhaber: Siemens Healthcare GmbH, 91052 Erlangen (DE)
(72) Erfinder: HAO, Peng, 90427 Nürnberg (DE); HÖLZER, Philipp, 08536 Plainsboro, NJ (US); IONASEC, Razvan, 90402 Nuernberg (DE)

(56) Entgegenhaltungen:
- US-A1- 2015 201 895
- WILLEMINK MARTIN J ET AL: "Finding the optimal dose reduction and iterative reconstruction level for coronary calcium scoring", JOURNAL OF CARDIOVASCULAR COMPUTED TOMOGRAPHY, ELSEVIER, AMSTERDAM, NL, Bd. 10, Nr. 1, 28. August 2015 (2015-08-28), Seiten 69-75, XP029388302, ISSN: 1934-5925, DOI: 10.1016/J.JCCT.2015.08.004
- NAKAZATO R ET AL: "Coronary artery calcium scoring using a reduced tube voltage and radiation dose protocol with dual-source computed tomography", JOURNAL OF CARDIOVASCULAR COMPUTED TOMOGRAPHY, ELSEVIER, AMSTERDAM, NL, Bd. 3, Nr. 6, 1. November 2009 (2009-11-01), Seiten 394-400, XP026808420, ISSN: 1934-5925 [gefunden am 2009-10-13]
- KENJI SUZUKI: "Pixel-Based Machine Learning in Medical Imaging", INTERNATIONAL JOURNAL OF BIOMEDICAL IMAGING, Bd. 4, Nr. 4, 1. Januar 2012 (2012-01-01), Seiten 166-18, XP055263162, ISSN: 1687-4188, DOI: 10.1155/2012/792079

## Beschreibung

Die Erfindung betrifft ein Verfahren zum Anpassen von Verfahrensschritten zur Ergebnisfindung in einem CT-basierten Entscheidungsunterstützungsverfahren an die Auswertung von LDCT-Bilddatensätzen. Weiterhin betrifft die Erfindung ein LDCT-basiertes Entscheidungsunterstützungsverfahren. Überdies betrifft die Erfindung eine Anpassungseinrichtung. Ferner betrifft die Erfindung ein System zur LDCT-basierten Entscheidungsunterstützung.

Verfahren der medizinischen Bildgebung werden auch im Rahmen von Früherkennungsprogrammen verwendet, um Krankheiten zu erkennen, bevor Symptome auftreten. Das Ziel solcher Untersuchungen besteht darin, Krankheiten zu einem frühestmöglichen Zeitpunkt in einem möglichst gut behandelbaren Stadium zu erkennen. Lungenkarzinome gehören zu den am schwierigsten zu behandelnden Krebsarten. Eine möglichst frühe Erkennung ist dabei notwendig, um das Behandlungsergebnis zu verbessern.

Ein dabei eingesetztes Bildgebungsverfahren ist die Computertomographie (kurz CT) des Brustbereichs. Die Computertomographie basiert auf der Erfassung von Röntgenstrahlung, wobei sogenannte Projektionsmessdaten erzeugt werden. Bei CT-Systemen läuft gewöhnlich eine an einer Gantry angeordnete Kombination aus Röntgenquelle und gegenüberliegend angeordnetem Röntgendetektor um einen Messraum um, in dem sich das Untersuchungsobjekt (das im Folgenden ohne Beschränkung der Allgemeinheit als Patient bezeichnet wird) befindet. Das Drehzentrum (auch "Isozentrum" genannt) fällt dabei mit einer sogenannten Systemachse z zusammen. Bei einem oder mehreren Umläufen wird der Patient mit Röntgenstrahlung der Röntgenquelle durchstrahlt, wobei mit Hilfe des gegenüberliegenden Röntgendetektors Projektionsmessdaten bzw. Röntgenprojektionsdaten erfasst werden. Auf Basis der Projektionsmessdaten werden dann Bilddaten rekonstruiert, welche dem Benutzer zur Begutachtung angezeigt werden.

Bei der Anwendung der Computertomographie zur Detektion von Lungenkrebs kann ein Tumor zu einem früheren Zeitpunkt erkannt werden als bei der herkömmlichen Brustradiographie. Bei der Früherkennung von Lungenkrebs werden Personen, welche ein erhöhtes Risiko für die Erkrankung an Lungenkrebs aufweisen, aber keine Symptome dieser Krankheit zeigen, mit Hilfe eines Niedrigdosis-CT-Bildgebungsverfahrens (kurz LDCT-Verfahren, LDCT = low dose CT) untersucht, wobei der Brustbereich abgebildet wird. Bei einem solchen LDCT-Verfahren werden Bilder mit ausreichender Qualität erzeugt, um viele Krankheiten im Brustbereich erkennen zu können, wobei bis zu 90 Prozent weniger an Dosisbelastung entsteht als bei einer herkömmlich Brust-CT-Bildgebung. Früherkennungsprogramme zur Erkennung von Lungenkrebs werden in den USA für Risikopersonen empfohlen. Das Risiko für Lungenkrebs hängt insbesondere von der Intensität der Rauchgewohnheiten einer Person, der Staubbelastung an deren Arbeitsplatz und deren Alter ab. Ähnliche Programme werden wohl in der Zukunft auch in Europa und Asien eingeführt werden.

Die LDCT-Thorax-Bilder, welche für die Früherkennung von Lungenkrebs erzeugt werden, weisen jedoch auch wertvolle Informationen bezüglich anderer Krankheiten auf. Es wäre also wünschenswert, diese Informationen zur Erkennung anderer Krankheiten des Thorax-Bereichs des Patienten zu verwenden und diesem somit zusätzliche CT-Untersuchungen zu ersparen.

Bis heute werden LDCT-Daten üblicherweise nur für die Früherkennung von Lungenkrebs verwendet, wobei es darum geht, Lungenknoten frühzeitig zu erkennen. Andere den Thorax betreffende Untersuchungen, wie zum Beispiel Gewebedichtemessungen, Lungenvolumenberechnungen, Messung des Volumens von Lungenknoten, Segmentierung der Lungenflügel, Untersuchungen der Atemwege, Messung der Knochendichte der Wirbel und sonstigen Knochen und Berechnungen des Calcium-Scores des Herzens, können nicht direkt auf LDCT-Daten angewandt werden, welche bei einer Bildgebung der Lunge für eine Lungenkrebsfrüherkennung erzeugt wurden. Diese LDCT-Daten könnten jedoch auch bei der Diagnose anderer Krankheiten, wie zum Beispiel COPD, interstitielle Lungenerkrankung, Erkrankung der Herzkranzgefäße und Osteoporose, zur Diagnose herangezogen werden oder sogar bei der Planung der Therapie der genannten Krankheiten eine Rolle spielen.

Insbesondere bei der Ermittlung eines Calcium-Scores und bei Messungen der Knochendichte werden einige dezidierte Verfahrensschritte bei der Bilddatenakquisition benötigt, wie zum Beispiel eine Akquisition eines Knochenphantoms bei der Knochendichteaufnahme oder die Anwendung einer Taktung einer Bildaufnahme mit einem Elektrokardiogramm bei einer Messung eines Calcium-Scores. Solche dezidierten Verfahrensschritte verhindern bisher die Wiederverwendung von LDCT-Daten bei diesen Studien. Außerdem lassen sich die bei normalen CT-Bildern zur Auswertung benutzten Verfahrensschritte nicht einfach unmodifiziert auf LDCT-Bilder übertragen.

In Willemink Martin J et al: "Finding the optimal dose reduction and iterative reconstruction level for coronary calcium scoring", Journal of Cardiovascular Computed Tomography, Elsevier, Amsterdam, NL, Bd. 10, Nr. 1, 28. August 2015 (2015-08-28), Seiten 69-75, XP029388302, ISSN: 1934-5925, DOI: 10.1016/J.JCCT.2015.08.004 ist die Ermittlung von Agatston-Scores auf der Basis einer LDCT-Bildgebung beschrieben. Dabei werden Bilddaten mit unterschiedlichen Röhrenströmen und Strahlendosen erzeugt und die auf deren Basis ermittelten Agatston-Scores werden mit Referenzdaten verglichen, um geeignete Parameter für LDCT-Aufnahmen zu finden.

Es besteht also das Problem, eine automatisierte Entscheidungsunterstützung, welche Fragestellungen zu einer Vielzahl von Erkrankungen im Thoraxbereich betreffen kann, auf der Basis von Bilddaten so zu gestalten, dass die dafür notwendigen Untersuchungen, möglichst zeitsparend, effektiv und schonend für den Patienten durchgeführt werden können und trotzdem eine ausreichende Qualität der entscheidungsunterstützenden Daten beibehalten wird.

Diese Aufgabe wird durch ein Verfahren zum Anpassen von Verfahrensschritten zur Ergebnisfindung in einem CT-basierten Entscheidungsunterstützungsverfahren an die Auswertung von LDCT-Bilddatensätzen gemäß Patentanspruch 1, ein LDCT-basiertes Entscheidungsunterstützungsverfahren gemäß Patentanspruch 9, eine Anpassungseinrichtung gemäß Patentanspruch 11 und ein System zur LDCT-basierten Entscheidungsunterstützung gemäß Patentanspruch 12 gelöst.

Bei dem erfindungsgemäßen Verfahren zum Anpassen von Verfahrensschritten zur Ergebnisfindung in einem CT-basierten Entscheidungsunterstützungsverfahren an die Auswertung von LDCT-Bilddatensätzen werden eine Mehrzahl von Referenz-Bilddatensätzen von einer Mehrzahl von Patienten erfasst. Dabei weist ein Referenz-Bilddatensatz jeweils mindestens einen CT-Bilddatensatz von einem der Mehrzahl von Patienten und zusätzlich einen LDCT-Bilddatensatz von diesem Patienten auf. Beispielsweise bilden beide Bilddatensätze einen gewissen Körperbereich, vorzugsweise denselben Körperbereich, von dem jeweiligen Patienten ab. Besonders bevorzugt umfasst der jeweils abgebildete Körperbereich den Thorax des jeweiligen Patienten. In jedem Fall bietet der CT-Bilddatensatz, welcher standardmäßig in einem CT-basierten Entscheidungsunterstützungsverfahren verwendet wird, eine Vergleichsmöglichkeit bei der Anpassung von Verfahrensschritten zur Ergebnisfindung an die Auswertung von LDCT-Bildern. Die Referenz-Bilddaten bilden eine Referenz-Datenbasis, welche zur Anpassung des CT-basierten Entscheidungsunterstützungsverfahrens an die Auswertung von LDCT-Bilddaten genutzt wird. Das erfindungsgemäße Verfahren basiert darauf, dass ein Entscheidungsunterstützungsverfahren zu einer oder mehreren medizinischen Fragestellungen, beispielsweise der Diagnose von Krankheiten, auf Basis von CT-Bilddaten verfügbar ist. Diese bereits bekannten Verfahrensschritte sollen nun zu denselben Fragenstellungen auf die Bearbeitung von LDCT-Bilddaten angepasst werden.

Weiterhin werden dann Verfahrensschritte zum Ermitteln von Ergebnisdaten auf die unterschiedlichen Bilddatensätze der Referenz-Bilddatensätze angewandt. D.h., es werden Verfahrensschritte eines vorbekannten CT-basierten Entscheidungsunterstützungsverfahrens nun sowohl auf die CT-Bilddaten als auch auf die LDCT-Bilddaten angewandt. Da die Verfahrensschritte für die CT-Bilddaten zuverlässig funktionieren, können nachfolgend die auf Basis der CT-Bilddaten gewonnenen Ergebnisdaten für einen Vergleich zur Überprüfung der Zuverlässigkeit der Verfahrensschritte bei der Auswertung von LDCT-Bilddaten verwendet werden. Als Ergebnisdaten sollen Daten verstanden werden, welche im Rahmen des Entscheidungsunterstützungsverfahrens zum Ziel der Ergebnisfindung auf Basis von ausgewerteten Bilddaten automatisiert ermittelt werden. Ergebnisdaten können zum Beispiel spezifischen, zu detektierenden Pathologien zugeordnet sein, welche bestimmten Krankheiten zugeordnet sind.

Wie bereits erwähnt, findet weiterhin ein Vergleich der Ergebnisdaten, welche auf Basis der CT-Bilddaten gewonnen wurden, und der Ergebnisdaten, welche auf Basis der LDCT-Bilddaten gewonnen wurden, statt. Vorteilhaft können nun Abweichungen bei der Ergebnisfindung auf Basis der LDCT-Bilddaten ermittelt werden. Diese Abweichungen werden im weiteren Verlauf des Verfahrens dazu genutzt, die Verfahrensschritte zum Ermitteln von Ergebnisdaten an das Ermitteln von Ergebnisdaten anhand eines LDCT-Bilddatensatzes anzupassen.

Auf diese Weise wird ein LDCT-basiertes Entscheidungsunterstützungsverfahren geschaffen, welches je nachdem, welche Arten von Ergebnisdaten bei dem Vergleich im Rahmen des Trainings bzw. der Anpassung herangezogen werden, für unterschiedliche medizinische Fragenstellungen genutzt werden kann.

Vorzugsweise wird das erfindungsgemäße Verfahren dazu genutzt, die Auswertung von LDCT-Bilddatensätzen bezüglich Ergebnisdaten zu trainieren, welche zur Entscheidungsunterstützung bei einer Diagnose einer anderen Krankheit als Lungenkrebs verwendet werden können. Wie bereits erläutert, war es bisher nur möglich, LDCT-Bilddaten zur Erkennung von Lungenkrebs zu verwenden. Mit dem erfindungsgemäßen Verfahren wird es nun ermöglicht, diese LDCT-Bilddaten auch automatisiert nach Anzeichen für andere Krankheiten auszuwerten. Auf diese Weise wird der Untersuchungsaufwand des Patienten reduziert. Außerdem kann aufgrund der Nutzung von LDCT-Bilddaten anstatt von CT-Bilddaten die Strahlendosis des Patienten vermindert werden. Eine Mehrfachverwendung der LDCT-Bilddaten ermöglicht es erst, eine Früherkennung einer Vielzahl von Krankheiten im Thoraxbereich in einem kurzen Zeitraum routinemäßig durchzuführen, ohne den Patienten durch eine zu hohe Strahlenbelastung unnötig zu gefährden. Die beschriebenen Verfahrensschritte werden vorzugsweise automatisiert ausgeführt, um die Anpassung unabhängig von den Fähigkeiten und Kenntnissen eines Benutzers zu machen und die Anpassung mit Hilfe einer größeren Datenbasis von Referenzdaten zeitsparend und effizient durchführen zu können. Die Verwendung einer größeren Datenbasis ermöglicht eine verbesserte Anpassung des Entscheidungsunterstützungsverfahrens an die Auswertung von LDCT-Bilddaten.

Bei dem erfindungsgemäßen LDCT-basierten Entscheidungsunterstützungsverfahren werden LDCT-Projektionsmessdaten von einem Patienten, vorzugsweise von dem Thorax eines Patienten, erfasst. Auf Basis der erfassten LDCT-Projektionsmessdaten werden LDCT-Bilddaten rekonstruiert. Weiterhin werden im Rahmen des erfindungsgemäßen LDCT-basierten Entscheidungsunterstützungsverfahren Verfahrensschritte zum Ermitteln von Ergebnisdaten, welche mit Hilfe des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen an die Verarbeitung von LDCT-Bilddaten angepasst wurden, auf die rekonstruierten LDCT-Bilddaten angewendet. Nach Anwendung des Entscheidungsunterstützungsverfahrens liegen Ergebnisdaten vor, auf deren Basis eine Information zur Unterstützung einer Diagnoseentscheidung an den Benutzer übermittelt wird. Vorteilhaft kann das erfindungsgemäße LDCT-basierten Entscheidungsunterstützungsverfahren wie schon bisher bekannte CT-basierte Entscheidungsunterstützungsverfahren auf verschiedene medizinische Fragestellungen, insbesondere auf die Suche nach unterschiedlichen Krankheitsphänomenen angewendet werden. Dabei kann die Auswertung hinsichtlich der unterschiedlichen Fragestellungen auf Basis ein- und desselben LDCT-Bilddatensatzes erfolgen, wodurch der Untersuchungsaufwand stark reduziert wird und die Dosisbelastung aufgrund der Verwendung eines Niedrigdosis-Bildgebungsverfahrens ebenfalls deutlich vermindert wird. Insbesondere bei Personen mit einer Vielzahl von Gesundheitsrisiken, beispielsweise starken Rauchern, kann das erfindungsgemäße Verfahren großen Nutzen bringen, da die genannten unterschiedlichen Gesundheitsrisiken mit einer geringeren Anzahl von Bildgebungsvorgängen, vorzugsweise nur einem einzigen niedrigdosierten LDCT-Bildgebungsvorgang, abgeklärt werden können, wodurch die Dosisbelastung des Patienten niedrig gehalten werden kann. Diese Verbesserung lässt die Anwendung einer Vielzahl von Früherkennungsmaßnahmen in den meisten Fällen erst gesundheitlich vertretbar erscheinen. Auch die beschriebenen Verfahrensschritte des Entscheidungsunterstützungsverfahrens werden vorzugsweise automatisiert ausgeführt, um die erzeugte Entscheidungsgrundlage unabhängig von den individuellen Ansichten, Fähigkeiten und Kenntnissen von Experten zu machen und die Auswertung der LDCT-Bilddaten zeitsparend und effizient durchführen zu können.

Die erfindungsgemäße Anpassungseinrichtung weist eine Eingangsschnittstelle zum Erfassen einer Mehrzahl von Referenz-Bilddatensätzen von einer Mehrzahl von Patienten auf. Ein Referenz-Bilddatensatz umfasst jeweils mindestens einen CT-Bilddatensatz von einem der Mehrzahl von Patienten und einen LDCT-Bilddatensatz von dem Patienten. Teil der erfindungsgemäßen Anpassungseinrichtung ist auch eine Ergebnisdaten-Ermittlungseinheit zum Anwenden von Verfahrensschritten zum Ermitteln von Ergebnisdaten auf die unterschiedlichen Bilddatensätze der Referenz-Bilddatensätze. Zudem umfasst die erfindungsgemäße Anpassungseinrichtung eine Vergleichseinheit zum Vergleichen der Ergebnisdaten miteinander. Außerdem weist die erfindungsgemäße Anpassungseinrichtung eine Anpassungseinheit zum Anpassen der Verfahrensschritte zum Ermitteln von Ergebnisdaten an das Ermitteln von Ergebnisdaten anhand eines LDCT-Bilddatensatzes auf Basis eines Ergebnisses des Vergleichs auf, wobei eine Anpassung an eine Mehrzahl von unterschiedlichen medizinischen Fragestellungen erfolgt.

Die Ergebnisdaten-Ermittlungseinheit kann zum Beispiel eine Parameterextraktionseinheit zum Anwenden eines Verfahrensschritts zur Parameterextraktion auf die unterschiedlichen Bilddatensätze der Referenz-Bilddatensätze umfassen. Bei der Parameterextraktion werden jeweils Parameterdatensätze gewonnen. Eine Ergebnisdaten-Berechnungseinheit, welche ebenfalls Teil der Ergebnisdaten-Ermittlungseinheit ist, kann dann zum Ermitteln von Ergebnisdaten auf Basis der extrahierten Parameterdatensätze eingesetzt werden.

Das erfindungsgemäße System zur LDCT-basierten Entscheidungsunterstützung weist eine LDCT-Bildgebungseinrichtung auf. Als LDCT-Bildgebungseinrichtung soll eine CT-Bildgebungseinrichtung verstanden werden, die zur Durchführung sogenannter LDCT-Bildaufnahmen geeignet ist. Die LDCT-Bildgebungseinrichtung ist dazu eingerichtet, LDCT-Projektionsmessdaten von einem Patienten zu erfassen. Weiterhin ist die LDCT-Bildgebungseinrichtung dazu eingerichtet, LDCT-Bilddaten auf Basis der erfassten LDCT-Projektionsmessdaten zu rekonstruieren. Teil des erfindungsgemäßen Systems zur LDCT-basierten Entscheidungsunterstützung ist auch eine Entscheidungsunterstützungseinrichtung, welche dazu eingerichtet ist, Verfahrensschritte zum Ermitteln von Ergebnisdaten, welche mit Hilfe eines Verfahrens nach einem der Ansprüche 1 bis 8 an die Verarbeitung von LDCT-Bilddaten angepasst wurden, auf die rekonstruierten LDCT-Bilddaten anzuwenden. Das erfindungsgemäße System zur LDCT-basierten Entscheidungsunterstützung umfasst auch eine Ausgabeeinheit zum Ausgeben einer Information zur Unterstützung einer Mehrzahl von Diagnoseentscheidungen. Die Information basiert auf den ermittelten Ergebnisdaten und kann im einfachsten Fall zum Beispiel die Ergebnisdaten umfassen.

Die wesentlichen Komponenten der erfindungsgemäßen Anpassungseinrichtung und Teile des Systems zur LDCT-basierten Entscheidungsunterstützung können zum überwiegenden Teil in Form von Softwarekomponenten ausgebildet sein. Dies betrifft insbesondere Teile der Ergebnisdaten-Ermittlungseinheit, der Vergleichseinheit und der Anpassungseinheit der Anpassungseinrichtung und Teile der Entscheidungsunterstützungseinrichtung. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher für medizinische Aufgaben genutzte verwendete Rechnersysteme auf einfache Weise durch ein Software-Update nachgerüstet werden können, um auf die erfindungsgemäße Weise als Anpassungseinrichtung und/oder Entscheidungsunterstützungseinrichtung zu arbeiten. Insofern wird die Aufgabe auch durch ein entsprechendes Computerprogrammprodukt mit einem Computerprogramm gelöst, welches direkt in eine Speichereinrichtung eines solchen Rechnersystem ladbar ist, mit Programmabschnitten, um alle Schritte des erfindungsgemäßen Verfahrens auszuführen, wenn das Computerprogramm in dem Rechnersystem ausgeführt wird.

Ein solches Computerprogrammprodukt kann neben dem Computerprogramm gegebenenfalls zusätzliche Bestandteile, wie z.B. eine Dokumentation und/oder zusätzliche Komponenten, auch Hardware-Komponenten, wie z.B. Hardware-Schlüssel (Dongles etc.) zur Nutzung der Software, umfassen
Zum Transport zur Speichereinrichtung des Rechnersystems und/oder zur Speicherung an dem Rechnersystem kann ein computerlesbares Medium, beispielsweise ein Memorystick, eine Festplatte oder ein sonstiger transportabler oder fest eingebauter Datenträger dienen, auf welchem die von einer Rechnereinheit einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren Beschreibungsteilen weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen erfolgt das Anpassen der Verfahrensschritte zum Ermitteln von Ergebnisdaten derart, dass die darauf basierenden Ergebnisdaten den Ergebnisdaten entsprechen, welche auf Basis des zugeordneten CT-Bilddatensatzes ermittelt wurden. Mit Hilfe der Referenz-Bilddaten wird also ein Entscheidungsunterstützungsverfahren auf das Verarbeiten von LDCT-Bilddaten hin so lange trainiert, bis die Ergebnisdaten auf Basis der LDCT-Bilddaten von den Ergebnisdaten, welche auf Basis der korrespondierenden CT-Bilddatensätze erzeugt wurden nicht mehr abweichen bzw. nicht mehr als um ein vorbestimmtes Maß abweichen. Wird der Vergleich mit einer genügend großen Datenbasis durchgeführt, so kann sichergestellt werden, dass die Ergebnisdaten bei der Auswertung von LDCT-Bilddaten korrekt sind.

In einer Ausgestaltung des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen umfassen die Verfahrensschritte zum Ermitteln von Ergebnisdaten die folgenden Schritte:
- Anwenden eines Verfahrensschritts zur Parameterextraktion auf die unterschiedlichen Bilddatensätze der Referenz-Bilddatensätze, wobei jeweils Parameterdatensätze gewonnen werden,
- Anwenden eines Verfahrensschritts zum Ermitteln von Ergebnisdaten auf Basis der extrahierten Parameterdatensätze.

Als Parameterextraktion soll in diesem Zusammenhang eine Ermittlung eines auf einem Bilddatensatz basierenden Parameters oder Parameterwerts verstanden werden. Ein solcher Parameter kann zum Beispiel eine Größe eines Knotens, eine Fläche oder ein Volumen und/oder eine Dichte einer Kalzifizierung von Gefäßstrukturen, Kontrastwerte, bei einer Segmentierung ermittelte Berandungslinien oder Volumengrößen umfassen. Auf Basis dieser extrahierten Parameter können dann Ergebnisdaten ermittelt werden. Derartige Ergebnisdaten umfassen Größen, welche Informationen umfassen, die bei der Behandlung einer medizinischen Fragestellung als Entscheidungshilfe genutzt werden können. Beispielsweise kann auf Basis der ermittelten Kalzifizierungen und deren Ausmaß ein Calcium-Score als Ergebnisdaten berechnet werden, der dann als Kriterium für ein Risiko eines Herzinfarkts dienen kann. Bilden zum Beispiel Läsionen die genannten Ergebnisdaten, so kann zum Beispiel die Schwelle für die Detektion einer Läsion geändert werden. Würde zum Beispiel bei der Auswertung von CT-basierten Bilddaten eine vollständig geschlossene Umrisslinie, wobei die Umrisslinie bzw. deren Verlauf den zu extrahierenden Parameter bildet, als notwendig für das Identifizieren einer Läsion betrachtet werden, so kann bei der Auswertung von LDCT-Bilddaten dieses Kriterium auf eine nur halb geschlossene Umrisslinie reduziert werden. Dadurch wird die etwas geringere Bildqualität der LDCT-Bilddaten kompensiert.

In einer Ausgestaltung des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen erfolgt die Parameterextraktion vorzugsweise derart, dass die darauf basierenden Ergebnisdaten den Ergebnisdaten entsprechen, welche auf Basis des zugeordneten CT-Bilddatensatzes ermittelt wurden. Anders ausgedrückt, erfolgt bei dieser Variante die Anpassung der Auswertung der LDCT-Bilddaten bereits bei dem Schritt der Gewinnung der Parameter, auf deren Basis dann die Ergebnisdaten gewonnen werden.

Bei dieser Variante wird also bereits der Vorgang der Parameterextraktion an die Verarbeitung von LDCT-Bilddatensätzen angepasst und zwar vorzugsweise derart, dass die auf Basis von LDCT-Bilddatensätzen von ein- und demselben Patienten extrahierten Parameter den Parametern entsprechen, welche auf der Basis von CT-Bilddaten von diesem Patienten ermittelt werden. Vorteilhaft müssen bei dieser Variante bei dem Auswertungsschritt zur Erzeugung von Ergebnisdaten auf Basis der extrahierten Parameter weniger Anpassungsprozesse zur Anpassung an LDCT-Bilddaten erfolgen. Beispielsweise kann eine solche Anpassung dahingehend erfolgen, dass auf Basis der LDCT-Bilddaten ermittelte Parameter bzw. Parameterwerte derart korrigiert werden, dass sie den auf Basis der CT-Bilddaten ermittelten Parametern vom Wert her entsprechen.

Anders ausgedrückt basiert das Anpassen des Verfahrensschritts zur Parameterextraktion zusätzlich auf einem Vergleich der extrahierten Parameter, welche auf Basis der unterschiedlichen Bilddatensätze gewonnen wurden. Beispiele für das Anpassen der Parameterextraktion kann zum Beispiel das Ändern der Sensitivität bei der Segmentierung eines Objekts, beispielsweise eine Läsion, sein. Beispielsweise kann der notwendige Kontrastunterschied zwischen Läsion und Umgebung reduziert werden. Eine andere Art der Anpassung betrifft eine Korrektur der ermittelten Parameterwerte.

Das Anpassen der Verfahrensschritte zum Ermitteln von Ergebnisdaten an das Ermitteln von Ergebnisdaten anhand eines LDCT-Bilddatensatzes kann sowohl das Anpassen des Verfahrensschritts zur Parameterextraktion an die Parameterextraktion aus einem LDCT-Bilddatensatz als auch das Anpassen des Verfahrensschritts zum Ermitteln von Ergebnisdaten an das Ermitteln von Ergebnisdaten anhand eines LDCT-Bilddatensatzes umfassen. Es kann also sowohl der Schritt der Parameterextraktion als auch die Auswertung zur Erzeugung der Ergebnisdaten entsprechend angepasst werden. Mithin ergeben sich vorteilhaft mehrere Adaptionsmöglichkeiten, um die Auswertung der LDCT-Bilddatensätze entsprechend anzupassen.

In einer bevorzugten Ausgestaltung des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen stellen die Ergebnisdaten eine Entscheidungshilfe bei der Diagnose mindestens einer der folgenden Krankheiten dar:
- Lungenemphysem,
- Atemwegserkrankungen,
- Interstitielle Lungenerkrankung,
- kardiovaskuläre Erkrankung,
- Osteoporose,
- Krebserkrankungen,
- Knochenverletzungen.

Bei der Akquisition der LDCT-Projektionsmessdaten für die genannten LDCT-Bilddaten können zum Beispiel Zinnfilter eingesetzt werden, mit denen bevorzugt niederenergetische Anteile durchgelassen werden. Mit diesen Zinnfiltern kann eine verbesserte Bildqualität und eine geringere Dosisbelastung erreicht werden.

Um für den Fall einer Herzbildgebung die Herzbewegung zu kompensieren, können zum Beispiel CT-Systeme mit verringerter minimal erreichbarer Rotationszeit pro Bildaufnahme bzw. Frame-Zeit verwendet werden, wodurch eine verbesserte Auflösung erreicht wird.

Die genannten Erkrankungen betreffen insbesondere den Thorax-Bereich eines Patienten. Wird nun auf Basis einer LDCT-Bildgebung eine Lungenkrebsuntersuchung durchgeführt, so können gleichzeig Informationen zu anderen Krankheitsbildern des Thorax gewonnen werden. Personen mit Risiken für Lungenkrebs, beispielsweise Raucher oder Personen, welche an besonders staubbelasteten Arbeitsplätzen tätig sind, weisen zusätzlich auch hohe Risiken für die anderen genannten Erkrankungen im Thorax-Bereich auf. Bei dieser Variante wird das Entscheidungsunterstützungsverfahren auf diese zusätzlichen Krankheitsbilder erweitert, so dass die Vorteile einer Früherkennung auch für diese Krankheiten genutzt werden können, ohne zusätzliche Bildaufnahmen durchführen zu müssen und damit einhergehende Strahlungsbelastungen in Kauf nehmen zu müssen.

In einer besonders bevorzugten Ausgestaltung des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen umfassen die Ergebnisdaten eine der folgenden Informationen:
- die Lungengewebsdichte,
- Bronchienwandanalysedaten,
- zu erkennende Gewebsmuster der Lunge,
- den Calcium-Score,
- das Myokardfett,
- die Myokardgröße,
- die Knochendichte,
- Texturmerkmale
- Knochenstrukturdaten.

Zur Gewinnung spezieller Ergebnisdaten sind bestimmte technische Anforderungen bei der Bildgebung zu erfüllen. Derartige Anforderungen sind zum Beispiel in Chapter 20, Thoracic Imaging Procedures", Computed Tomographie for Technologists 2010, A Comprehensive Text, beschrieben.

Bei der Messung der Knochendichte kann zum Beispiel vorab ein Kalibrationsverfahren mit einem Kalibrationsphantom oder einer andersgearteten Referenzmessung eingesetzt werden, um Abschwächungswerte unabhängig von Variationen der Messbedingungen, wie zum Beispiel Eigenschaften der Scaneinheiten oder der Patientengeometrie zu machen. Eine solche Vorgehensweise ist zum Beispiel in US 2014/0 376 701 A1 beschrieben.

Für die Ermittlung von Texturmerkmalen können Detektoren mit einer erhöhten räumlichen Auflösung eingesetzt werden. Beispielsweise eignen sich photonenzählende Detektoren für eine höhere Auflösung besonders gut, da bei diesen die Auflösung nicht durch sogenannte Septen reduziert wird.

Um Strukturen besser sichtbar zu machen, können vor der Aufnahme von Projektionsmessdaten zur Erzeugung der LDCT-Bilddatensätze auch Kontrastmittel verabreicht werden.

Vorteilhaft können bei Anwendung der genannten zusätzlichen Maßnahmen bei der Durchführung von LDCT-Bildaufnahmen simultan Informationen bezüglich einer Vielzahl von Krankheitsbildern im Thoraxbereich mit verbesserter Qualität gewonnen werden, ohne zusätzliche Bildaufnahmen, insbesondere hochdosierte CT-Bildaufnahmen durchführen zu müssen.

In einer besonders sinnvoll anwendbaren Variante des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen erfolgt das Anpassen der Verfahrensschritte zum Ermitteln von Ergebnisdaten an das Ermitteln von Ergebnisdaten anhand eines LDCT-Bilddatensatzes mit Hilfe eines maschinellen Lernverfahrens. Maschinelles Lernen kann dazu genutzt werden, den Anpassungsvorgang der Verfahrensschritte zum Ermitteln von Ergebnisdaten an die Auswertung von LDCT-Bilddaten zu automatisieren. Der Anpassungsvorgang an die Verarbeitung von LDCT-Bilddatensätzen kann dabei als eine Art Trainingsverfahren gestaltet werden, bei dem auf Basis einer Trainingsdatenbasis ein schrittweises Annähern an eine korrekte Auswertung von LDCT-Bilddaten erfolgt. Eine besondere Art des maschinellen Lernens wird durch sogenannte Mehrschichtverfahren realisiert. Solche Verfahren werden zum Beispiel in Y. LeCun et al "Deep learning", nature review, doi: 10.1038/nature14539 oder in M. N. Wernick et al. "Machine Learning in Medical Imaging", IEEE signal processing magazine, July 2010 beschrieben.

In einer besonders bevorzugten Ausgestaltung des erfindungsgemäßen Verfahrens zum Anpassen von Verfahrensschritten zur Ergebnisfindung in einem CT-basierten Entscheidungsunterstützungsverfahren an die Auswertung von LDCT-Bilddatensätzen wird für den Fall, dass nach einem Anpassen der Verfahrensschritte zum Ermitteln von Ergebnisdaten an das Ermitteln von Ergebnisdaten anhand eines LDCT-Bilddatensatzes auf Basis der CT-Bilddaten und der LDCT-Bilddaten teilweise unterschiedliche Ergebnisdaten erzeugt werden, ein Wert für eine Wahrscheinlichkeit für eine korrekte Ermittlung von Ergebnisdaten auf Basis von LDCT-Bilddaten ermittelt. D.h., lässt sich das CT-basierte Entscheidungsunterstützungsverfahren nicht vollständig an die Auswertung von LDCT-Bilddaten anpassen, wobei auch nach der Anpassung eine gewisse Unsicherheit bei der Erzeugung von Ergebnisdaten verbleibt, so werden den Ergebnisdaten auch Informationen über diese Unsicherheit beigefügt, so dass ein Benutzer zum Beispiel entscheiden kann, ob er sich noch zusätzliche Informationen, insbesondere noch zusätzliche Bilddaten, beispielsweise CT-Bilddaten, verschafft, um eine sichere Datenbasis für seine Diagnose zu bekommen.

In einer Variante des erfindungsgemäßen LDCT-basierten Entscheidungsunterstützungsverfahren werden Protokollparameter für das Erfassen von LDCT-Projektionsmessdaten an mindestens eine zu diagnostizierende Krankheit angepasst. Als Protokollparameter sollen in diesem Zusammenhang Parameter eines Messprotokolls verstanden werden, mit dem ein Bildgebungsvorgang an individuelle Anforderungen angepasst werden kann. Beispielsweise kann für die Darstellung bestimmter Untersuchungsbereiche zu bestimmten Zwecken ein besonders starker Kontrast oder eine besonders starke zeitliche Auflösung gewählt werden, um Anforderung an eine Informationsqualität zu erfüllen.

Bei der Abbildung der Herzgefäße kann zum Beispiel durch eine Verkürzung der Frame-Zeit die zeitliche Auflösung verbessert und die Akquisitionszeit derart verkürzt werden, dass eine Art Einfrieren der Herzbewegung erreicht wird. Auf diese Weise kann eine Herzbildgebung mit einer Untersuchung eines anderen Thorax-Bereichs, beispielsweise der Lunge, kombiniert werden. Mithin kann auf eine EKG-getaktete, zusätzliche Bildaufnahme des Herzens verzichtet werden.

Die Erfindung wird im Folgenden unter Hinweis auf die beigefügten Figuren anhand von Ausführungsbeispielen noch einmal näher erläutert. Es zeigen:
FIG 1 ein Flussdiagramm, welches ein Verfahren zum Anpassen einer Parameterextraktion eines CT-basierten Entscheidungsunterstützungsverfahrens an die Auswertung von LDCT-Bilddatensätzen gemäß einem Ausführungsbeispiel der Erfindung veranschaulicht,
FIG 2 ein Flussdiagramm, welches ein LDCT-basiertes Entscheidungsunterstützungsverfahren gemäß einem Ausführungsbeispiel der Erfindung veranschaulicht,
FIG 3 ein Blockdiagramm, mit dem eine Anpassungseinrichtung gemäß einem Ausführungsbeispiel der Erfindung dargestellt wird,
FIG 4 ein Blockdiagramm, welches eine Einrichtung zur LDCT-basierten Entscheidungsunterstützung gemäß einem Ausführungsbeispiel der Erfindung umfasst.

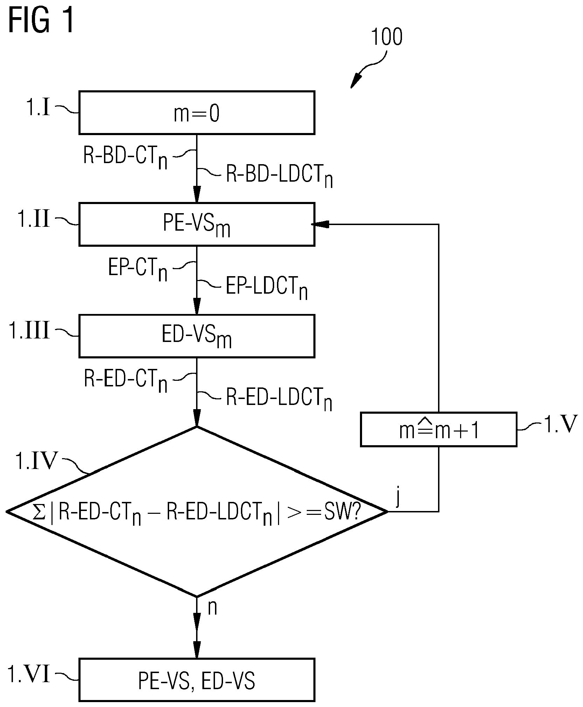

In FIG 1 ist ein Verfahren zum Anpassen eines CT-basierten Entscheidungsunterstützungsverfahrens an die Auswertung von LDCT-Bilddatensätzen veranschaulicht.

Bei dem Schritt 1.I werden zunächst aus einer Datenbank eine Mehrzahl von Referenz-Bilddatensätzen R-BD₁, ..., R-BDₙ,..., R-BD_{N} von einer Mehrzahl von Patienten P₁, ..., Pₙ, ..., P_{N} erfasst. Ein solcher Referenz-Bilddatensatz R-BDₙ umfasst jeweils sowohl einen CT-Bilddatensatz R-BD-CTₙ als auch einen entsprechenden LDCT-Bilddatensatz R-BD-LDCTₙ eines Patienten Pₙ. Beide Referenz-Sub-Bilddatensätze R-BD-CTₙ, R-BD-LDCTₙ sind jeweils von dem Brustbereich des Patienten Pₙ aufgenommen. Zu den Referenz-Bilddatensätzen R-BDₙ sind die bei der Auswertung der CT-Bilddatensätze R-BD-CTₙ verwendeten Parameterextraktionsverfahrensschritte PE-VS₀ und die Verfahrensschritte ED-VS₀ zur Ergebnisdatenbildung bekannt. Weiterhin sind auch die bei einem jeweils auf die CT-Bilddatensätze BD-CTₙ angewandten CT-basierten Entscheidungsunterstützungsverfahren ermittelten Parameterdaten EP-CTₙ und Ergebnisdaten R-ED-CTₙ bekannt. D.h., das in FIG 1 veranschaulichte Verfahren basiert darauf, dass ein Entscheidungsunterstützungsverfahren zu einer oder mehreren medizinischen Fragestellungen, beispielsweise der Diagnose von Krankheiten, auf Basis von CT-Bilddaten verfügbar ist.

Bei dem Schritt 1.II werden nun mit Hilfe der durch die Anwendung auf die CT-Bilddatensätze R-BD-CTₙ bereits bekannten Parameterextraktionsverfahrensschritte PE-VS₀ Parameterdaten EP-LDCTₙ aus den jeweiligen LDCT-Bilddatensätzen R-BD-LDCTₙ extrahiert. Bei einem solchen Parameter kann es sich zum Beispiel um Randlinien abgeschatteter Bereiche im Bildaufnahmevolumen oder ähnliches handeln. Ein solcher Parameter kann je nach dem zu untersuchenden Phänomen bzw. der damit korrelieren medizinischen Fragestellung sehr unterschiedlichen sein. Auf Basis der extrahierten Parameterdaten EP-LDCTₙ werden nachfolgend bei dem Schritt 1.III Ergebnisdaten R-ED-LDCTₙ ermittelt. Die Ergebnisdaten R-ED-LDCTₙ können zum Beispiel ein Auswertungsergebnis einer entscheidungsrelevanten Größe umfassen. Beispielsweise können auf Basis der mit Hilfe der Parameterextraktionsverfahrensschritte PE-VS₀ ermittelten abgeschatteten Flächen im Lungenbereich als Auswertungsergebnis Rückschlüsse auf die Größe von Lungenknoten gezogen werden, auf deren Basis eine Entscheidungshilfe für eine Diagnose gegeben werden kann.

Vorteilhaft wird sich bei den Schritten 1.II und 1.III auf die Extraktion von Parameterdaten bzw. auf die Ermittlung von Ergebnisdaten konzentriert, welche für eine oder mehrere vorbestimmte medizinische Fragestellungen relevant sind. Mithin kann ein möglicherweise für eine bestimmte Krankheit, beispielsweise Lungenkrebs, bereits geeignetes LDCT-Bildddatenbasiertes Entscheidungsunterstützungsverfahren an die Unterstützung der Diagnose anderer Krankheiten, welche den Thoraxbereich betreffen, angepasst werden.

Die auf Basis der LDCT-Bilddaten R-BD-LDCTₙ ermittelten Ergebnisdaten R-ED-LDCTₙ werden bei dem Schritt 1. IV mit den vorbekannten, auf den entsprechenden CT-Bilddaten R-BD-CTₙ beruhenden Ergebnisdaten R-ED-CTₙ verglichen. Für den Fall, dass die Gesamtheit der ermittelten, auf den LDCT-Bilddaten R-BD-LDCTₙ beruhenden Ergebnisdaten R-ED-LDCTₙ zu stark von der Gesamtheit der entsprechenden Ergebnisdaten R-ED-CTₙ, welche auf Basis der CT-Bilddaten R-BD-CTₙ gewonnen wurden, abweichen, was in FIG 1 mit "j" gekennzeichnet wird, erfolgt bei dem Schritt 1.V eine Anpassung der Parameterextraktionsverfahrensschritte PE-VS₀ und der Schritte ED-VS₀ zur Erzeugung von Ergebnisdaten, welche auf die LDCT-Bilddaten RE-LDCTₙ angewandt wurden. Die Anpassung kann zum Beispiel im Rahmen eines maschinellen Lernverfahrens erfolgen.

Mit derart angepassten Parameterextraktionsverfahrensschritten PE-VS₁ werden dann bei dem Schritt 1.II erneut Parameterdaten EP-LDCTₙ aus den jeweiligen LDCT-Bilddatensätzen R-BD-LDCTₙ extrahiert und es werden erneut bei dem Schritt 1.III Ergebnisdaten RE-LDCTₙ auf Basis der extrahierten Parameterdaten EP-LDCTₙ erzeugt. Die hierzu notwendigen Verfahrensschritte ED-VS₁ zur Ergebnisdatenbildung können ebenfalls bei dem Schritt 1.V durch Anpassung modifiziert worden sein. Anschließend erfolgt bei dem Schritt 1.IV erneut ein Vergleich der auf Basis der LDCT-Bildddaten R-BD-LDCTₙ erzeugten Ergebnisdaten R-ED-LDCTₙ mit den entsprechenden Ergebnisdaten R-ED-CTₙ, welche auf Basis der CT-Bilddatensätze R-BD-CTₙ bekannt sind. Wird bei dem Schritt 1.IV ermittelt, dass die Gesamtheit der auf Basis der LDCT-Bilddaten R-BD-LDCTₙ ermittelten Ergebnisdaten R-ED-LDCTₙ von der Gesamtheit der Ergebnisdaten R-ED-CTₙ, welche den entsprechenden CT-Bilddaten R-BD-CTₙ zugeordnet sind, nicht mehr als um ein vorbestimmtes Maß SW abweichen, was in FIG 1 mit "n" gekennzeichnet ist, so wird zu dem Schritt 1.VI übergegangen, bei dem die zuletzt verwendeten, angepassten Parameterextraktionsverfahrensschritte PE-VSₘ und die zuletzt verwendeten, angepassten Verfahrensschritte ED-VSₘ zur Ergebnisdatenbildung als für eine Auswertung von LDCT-Bilddaten geeignete Parameterextraktionsverfahrensschritte PE-VS bzw. Verfahrensschritte ED-VS zur Ergebnisdatenbildung festgelegt werden. Die so festgelegten Verfahrensschritte können nachfolgend bei einem Verfahren LDCT-basierten Entscheidungsunterstützungsverfahren eingesetzt werden. Je nach Art des Vergleichsmaterials, d.h. insbesondere der extrahierten Parameter und Ergebnisdaten der CT-Bilddaten können entsprechende angepasste Parameterextraktionsverfahrensschritte PE-VS und Verfahrensschritte ED-VSₘ zum Ermitteln von Ergebnisdaten für die LDCT-Bildgebung erzeugt werden. Damit lassen sich dann entsprechend angepasste LDCT-basierte Entscheidungsunterstützungsverfahren realisieren, mit deren Hilfe neben Lungenkrebs auch andere den Thorax betreffende Krankheiten diagnostiziert werden können.

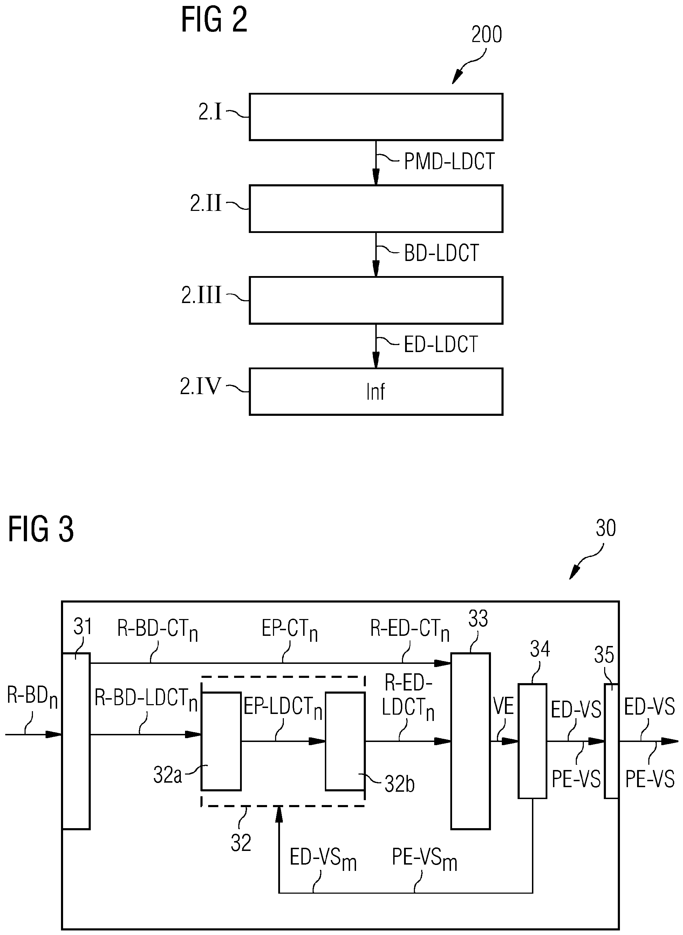

In FIG 2 ist ein Flussdiagramm 200 gezeigt, welches ein LDCT-basiertes Entscheidungsunterstützungsverfahren gemäß einem Ausführungsbeispiel der Erfindung veranschaulicht.

Bei dem Schritt 2.I werden zunächst mit Hilfe eines Computertomographiesystems LDCT-Projektionsmessdaten PMD-LDCT von dem Thorax eines Patienten P erfasst. Anschließend werden bei dem Schritt 2.II auf Basis der erfassten LDCT-Projektionsmessdaten PMD-LDCT LDCT-Bilddaten BD-LDCT rekonstruiert. Bei dem Schritt 2.III werden dann Verfahrensschritte PE-VS, ED-VS zum Ermitteln von Ergebnisdaten ED-LDCT auf die rekonstruierten Bilddaten BD-LDCT angewendet. Die Verfahrensschritte PE-VS, ED-VS wurden mit Hilfe des in FIG 1 veranschaulichten Verfahrens an die Verarbeitung von LDCT-Bilddaten BD-LDCT angepasst. Schließlich wird bei dem Schritt 2.IV eine Information Inf zur Unterstützung einer Diagnoseentscheidung auf Basis der ermittelten Ergebnisdaten ED-LDCT an einen Benutzer ausgegeben.

In FIG 3 ist eine Anpassungseinrichtung 30 gemäß einem Ausführungsbeispiel der Erfindung veranschaulicht. Eine solche Anpassungseinrichtung 30 umfasst eine Eingangsschnittstelle 31. Die Eingangsschnittstelle 31 ist dazu eingerichtet, eine Mehrzahl von Referenz-Bilddatensätzen R-BDₙ von einer Mehrzahl von Patienten Pₙ zu empfangen. Die Referenz-Bilddatensätze R-BDₙ umfassen jeweils einen CT-Bilddatensatz R-BD-CTₙ von einem der Mehrzahl von Patienten und einen LDCT-Bilddatensatz R-BD-LDCTₙ von dem Patienten Pₙ. Teil der Anpassungseinrichtung 30 ist auch eine Ergebnisdaten-Ermittlungseinheit 32. Die Ergebnisdaten-Ermittlungseinheit 32 ist dazu eingerichtet, auf Basis der LDCT-Bilddatensätze R-BD-LDCTₙ Ergebnisdaten R-ED-LDCTₙ zu erzeugen. Die Referenz-Bilddatensätze R-BDₙ umfassen zusätzlich zu dem CT-Bilddatensatz R-BD-CTₙ auch entsprechende Ergebnisdaten R-ED-CTₙ und Parameterdatensätze EP-CTₙ, welche vorab aus den CT-Bilddatensätzen R-BD-CTₙ gewonnen wurden.

Die Ergebnisdaten-Ermittlungseinheit 32 umfasst eine Parameterextraktionseinheit 32a zum Anwenden eines Verfahrensschritts zur Parameterextraktion auf die LDCT-Bilddatensätze R-BD-LDCTₙ der Referenz-Bilddatensätze R-BDₙ, wobei jeweils Parameterdatensätze EP-LDCTₙ gewonnen werden. Auf Basis der Parameterdatensätze EP-LDCTₙ werden dann von einer Ergebnisdaten-Berechnungseinheit 32b Ergebnisdaten R-ED-LDCTₙ rechnerisch ermittelt.

Die ermittelten Ergebnisdaten R-ED-LDCTₙ, welche auf den LDCT-Bilddaten R-BD-LDCTₙ beruhen, werden ebenso wie die vorab bekannten Ergebnisdaten R-ED-CTₙ, welche auf den CT-Bilddaten R-BD-CTₙ beruhen, an eine Vergleichseinheit 33 übermittelt, die dazu eingerichtet ist, Ergebnisdaten R-ED-CTₙ, R-ED-LDCTₙ miteinander zu vergleichen.

Von der Vergleichseinheit 33 wird dann ein Vergleichsergebnis VE an eine Anpassungseinheit 34 übermittelt, die dazu eingerichtet ist, Verfahrensschritte PE-VS₀, ED-VS₀ zum Ermitteln von Ergebnisdaten an das Ermitteln von Ergebnisdaten ED-LDCTₙ anhand eines LDCT-Bilddatensatzes BD-LDCTₙ, R-BD-LDCTₙ auf Basis des Vergleichsergebnisses VE anzupassen. Die angepassten Verfahrensschritte PE-VSₘ, ED-VSₘ können nachfolgend mit Hilfe der Ergebnisdaten-Ermittlungseinheit 32 und der Vergleichseinheit 33 getestet werden. Erfüllt das von der Vergleichseinheit 33 ermittelte Vergleichsergebnis vorbestimmte Qualitätskriterien, d.h., weichen die Ergebnisdaten ED-CTn, ED-LDCTₙ nur noch maximal um einen vorbestimmten Schwellwert voneinander ab, so werden von der Anpassungseinheit 34 die zuletzt ermittelten angepassten Verfahrensschritte PE-VS, ED-VS über eine Ausgabeschnittstelle 35 ausgegeben.

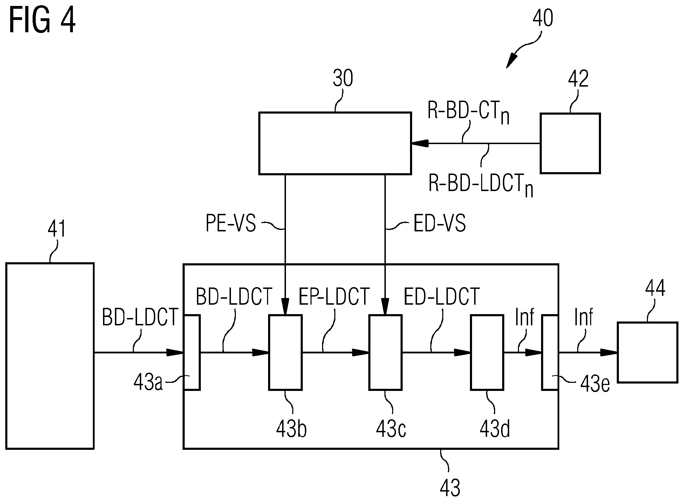

In FIG 4 ist ein System 40 zur LDCT-basierten Entscheidungsunterstützung gemäß einem Ausführungsbeispiel der Erfindung schematisch dargestellt. Das System 40 umfasst eine LDCT-Bildgebungseinrichtung 41, eine Datenbank 42, eine Entscheidungsunterstützungseinrichtung 43, eine Anzeigeeinheit 44 und eine der in FIG 3 veranschaulichten Anordnung 30 entsprechende Anpassungseinrichtung 30. Mit Hilfe der LDCT-Bildgebungseinrichtung 41 werden zunächst Projektionsmessdaten PMD-LDCT von einem Patienten akquiriert. Auf Basis der Projektionsmessdaten werden LDCT-Bilddaten BD-LDCT rekonstruiert und an die Entscheidungsunterstützungseinrichtung 43 übermittelt. Dort werden die LDCT-Bilddaten BD-LDCT von einer Eingangsschnittstelle 43a empfangen und an eine Parameterextraktionseinheit 43b weitergegeben, welche dazu eingerichtet ist, Verfahrensschritte EP-VS zur Parameterextraktion auf die LDCT-Bilddatensätze BD-LDCTₙ anzuwenden, wobei Parameterdatensätze EP-LDCT gewonnen werden. Auf Basis der Parameterdatensätze EP-LDCT werden dann von einer Ergebnisdaten-Berechnungseinheit 43c Ergebnisdaten ED-LDCT rechnerisch ermittelt und an eine Einheit 43d zur Erzeugung von Informationsdaten Inf übermittelt. Die Parameterextraktionseinheit 43b und die Ergebnisdaten-Berechnungseinheit 43c erhalten jeweils von der Anpassungseinrichtung 30 speziell an die Verarbeitung von LD-CT-Bilddaten angepasste Verfahrensschrittprozeduren PE-VS, ED-VS, mit denen die genannten Parameterdatensätze EP-LDCT und Ergebnisdaten ED-LDCT ermittelt werden. Die von der Einheit 43d zur Erzeugung von Informationsdaten Inf erzeugten Informationen Inf werden anschließend über eine Ausgabeschnittstelle 43e an die als Ausgabeeinheit diendende Anzeigeeinheit 44 ausgegeben. Ein Benutzer kann sich dann die dort angezeigten Informationen als Entscheidungshilfe für eine Diagnose oder eine weitere Behandlung eines Patienten ansehen. Die Anpassungseinrichtung 30 ist mit der erwähnten Datenbank 42 verbunden, welche Referenzbilddaten R-BDₙ gespeichert hat, welche jeweils korrespondierende Referenz-CT-Bilddatensätze R-BD-CTₙ und Referenz-LDCT-Bilddatensätze R-BD-LDCTₙ aufweisen.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. So wurde das Anpassungsverfahren in erster Linie in Bezug auf eine Anwendung im Bereich der Lunge beschrieben. Die Erfindung ist jedoch nicht auf die konkrete Anwendung beschränkt, sondern die Erfindung kann auch grundsätzlich auf eine Vielzahl von unterschiedlichen, den Brustbereich betreffenden medizinischen Fragestellungen angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zum Anpassen von Verfahrensschritten zur Ergebnisfindung in einem CT-basierten Entscheidungsunterstützungsverfahren an die Auswertung von LDCT-Bilddatensätzen (BD-LDCTₙ), aufweisend die Schritte:
- Erfassen einer Mehrzahl von Referenz-Bilddatensätzen (R-BDₙ) von einer Mehrzahl von Patienten, wobei ein Referenz-Bilddatensatz (R-BDₙ) jeweils mindestens aufweist:
- einen CT-Bilddatensatz (R-BD-CTₙ) von einem (Pₙ) der Mehrzahl von Patienten,
- einen LDCT-Bilddatensatz (R-BD-LDCTₙ) von dem Patienten (Pn),
- Anwenden von Verfahrensschritten (PE-VS₀, ED-VS₀) zum Ermitteln von Ergebnisdaten (ED-CT, ED-LDCT) auf die unterschiedlichen Bilddatensätze (R-BD-CTₙ, R-BD-LDCTₙ) der Referenz-Bilddatensätze (R-BDₙ),
- Vergleichen der Ergebnisdaten (R-ED-CTₙ, R-ED-LDCTₙ) miteinander,
- Anpassen der Verfahrensschritte (PE-VS₀, ED-VS₀) zum Ermitteln von Ergebnisdaten (ED-CT, ED-LDCT) an das Ermitteln von Ergebnisdaten (ED-LDCT) anhand eines LDCT-Bilddatensatzes (BD-LDCTₙ, R-BD-LDCTₙ) auf Basis eines Ergebnisses (VE) des Vergleichs,
wobei eine Anpassung an eine Mehrzahl von unterschiedlichen medizinischen Fragestellungen erfolgt.

2. Verfahren nach Anspruch 1, wobei die Verfahrensschritte (PE-VS₀, ED-VS₀) zum Ermitteln von Ergebnisdaten (R-ED-CTₙ, R-ED-LDCTₙ) die folgenden Schritte umfassen:
- Anwenden eines Verfahrensschritts (PE-VS₀) zur Parameterextraktion auf die unterschiedlichen Bilddatensätze (R-BD-CTₙ, R-BD-LDCTₙ) der Referenz-Bilddatensätze (R-BDₙ), wobei jeweils Parameterdatensätze (EP-CTₙ, EP-LDCTₙ) gewonnen werden,
- Anwenden eines Verfahrensschritts (ED-VS₀) zum Ermitteln von Ergebnisdaten (R-ED-CTₙ, RE-LDCTₙ) auf Basis der extrahierten Parameterdatensätze (EP-CTₙ, EP-LDCTₙ).

3. Verfahren nach Anspruch 2, wobei das Anpassen der Verfahrensschritte (EP-VS₀, ED-VS₀) zum Ermitteln von Ergebnisdaten (ED-CT, ED-LDCT) an das Ermitteln von Ergebnisdaten (ED-LDCT) anhand eines LDCT-Bilddatensatzes (BD-LDCTₙ, R-BD-LDCTₙ)
- das Anpassen des Verfahrensschritts (EP-VS₀) zur Parameterextraktion an die Parameterextraktion aus einem LDCT-Bilddatensatz (R-BD-LDCTₙ) und/oder
- das Anpassen des Verfahrensschritts (ED-VS₀) zum Ermitteln von Ergebnisdaten (ED-CT, ED-LDCT) an das Ermitteln von Ergebnisdaten (ED-LDCTₙ) anhand eines LDCT-Bilddatensatzes (R-BD-LDCTₙ) umfasst.

4. Verfahren nach einem der vorstehenden Ansprüche, wobei die Ergebnisdaten (ED-CT, ED-LDCT) eine Entscheidungshilfe bei der Diagnose mindestens einer der folgenden Krankheiten darstellen:
- Lungenemphysem,
- Atemwegserkrankungen,
- Interstitielle Lungenerkrankung,
- kardiovaskuläre Erkrankung,
- Osteoporose,
- Krebserkrankungen,
- Knochenverletzungen.

5. Verfahren nach einem der Ansprüche 2 bis 4, wobei das Anpassen des Verfahrensschritts zur Parameterextraktion zusätzlich auf einem Vergleich der extrahierten Parameter (EP-CTₙ, EP-LDCTₙ), welche auf Basis der unterschiedlichen Bilddatensätze (R-BD-CTₙ, R-BD-LDCTₙ) gewonnen wurden, basiert.

6. Verfahren nach einem der Ansprüche 2 bis 5, wobei die extrahierten Parameterdatensätze (EP-CTₙ, EP-LDCTₙ) eine der folgenden Parameterarten umfassen:
- die Lungengewebsdichte,
- Bronchienwandanalysedaten,
- zu erkennende Gewebsmuster der Lunge,
- einen Calcium-Score,
- das Myokardfett,
- die Myokardgröße,
- die Knochendichte,
- Texturmerkmale
- Knochenstrukturdaten.

7. Verfahren nach einem der vorstehenden Ansprüche, wobei das Anpassen der Verfahrensschritte (EP-VS₀, ED-VS₀) zum Ermitteln von Ergebnisdaten (ED-CT, ED-LDCT) an das Ermitteln von Ergebnisdaten (ED-LDCT) anhand eines LDCT-Bilddatensatzes (BD-LDCTₙ, R-BD-LDCTₙ) mit Hilfe eines maschinellen Lernverfahrens erfolgt.

8. Verfahren nach einem der vorstehenden Ansprüche, wobei für den Fall, dass nach einem Anpassen der Verfahrensschritte (EP-VS₀, ED-VS₀) zum Ermitteln von Ergebnisdaten (ED-CT, ED-LDCT) an das Ermitteln von Ergebnisdaten (ED-LDCT) anhand eines LDCT-Bilddatensatzes (BD-LDCTₙ, R-BD-LDCTₙ) auf Basis der CT-Bilddaten und der LDCT-Bilddaten teilweise unterschiedliche Ergebnisdaten (R-ED-CTₙ, R-ED-LDCTₙ) erzeugt werden, ein Wert für eine Wahrscheinlichkeit für eine korrekte Ermittlung von Ergebnisdaten (R-ED-LDCT) auf Basis von LDCT-Bilddaten (BD-LDCT) ermittelt wird.

9. LDCT-basiertes Entscheidungsunterstützungsverfahren, aufweisend die Schritte:
- Erfassen von LDCT-Projektionsmessdaten (LDCT-PMD) von einem Patienten,
- Rekonstruieren von LDCT-Bilddaten (LDCT-BD) auf Basis der erfassten LDCT-Projektionsmessdaten (LDCT-PMD),
- Anwenden von Verfahrensschritten zum Ermitteln von Ergebnisdaten (ED-LDCT), welche mit Hilfe eines Verfahrens nach einem der Ansprüche 1 bis 8 an die Verarbeitung von LDCT-Bilddaten (LDCT-BD) angepasst wurden, auf die rekonstruierten LDCT-Bilddaten (LDCT-BD)
- Ausgeben einer Information zur Unterstützung einer Mehrzahl von Diagnoseentscheidungen auf Basis der ermittelten Ergebnisdaten (ED-LDCT).

10. Verfahren nach Anspruch 9, wobei Protokollparameter für das Erfassen von LDCT-Projektionsmessdaten (LDCT-PMD) an mindestens eine zu diagnostizierende Krankheit angepasst werden.

11. Anpassungseinrichtung (30), aufweisend:
- eine Eingangsschnittstelle (31) zum Erfassen einer Mehrzahl von Referenz-Bilddatensätzen (R-BDₙ) von einer Mehrzahl von Patienten, wobei ein Referenz-Bilddatensatz (R-BDₙ) jeweils mindestens aufweist:
- einen CT-Bilddatensatz (R-BD-CTₙ) von einem (Pn) der Mehrzahl von Patienten,
- einen LDCT-Bilddatensatz (R-BD-LDCTₙ) von dem Patienten (Pn),
- eine Ergebnisdaten-Ermittlungseinheit (32) zum Anwenden von Verfahrensschritten (EP-VS₀, ED-VS₀) zum Ermitteln von Ergebnisdaten (R-ED-CTₙ, R-ED-LDCTₙ) auf die unterschiedlichen Bilddatensätze (R-BD-CTₙ, R-BD-LDCTₙ) der Referenz-Bilddatensätze (R-BDₙ),
- eine Vergleichseinheit (33) zum Vergleichen der Ergebnisdaten (R-ED-CTₙ, R-ED-LDCTₙ) miteinander,
- eine Anpassungseinheit (34) zum Anpassen der Verfahrensschritte (EP-VS₀, ED-VS₀) zum Ermitteln von Ergebnisdaten (R-ED-CTₙ, R-ED-LDCTₙ) an das Ermitteln von Ergebnisdaten (ED-LDCT) anhand eines LDCT-Bilddatensatzes (BD-LDCT, BD-LDCT) auf Basis eines Ergebnisses (VE) des Vergleichs, wobei eine Anpassung an eine Mehrzahl von unterschiedlichen medizinischen Fragestellungen erfolgt.

12. System (40) zur LDCT-basierten Entscheidungsunterstützung, aufweisend:
- eine LDCT-Bildgebungseinrichtung (41) zum Erfassen von LDCT-Projektionsmessdaten (PMD-LDCT) von einem Patienten und zum Rekonstruieren von LDCT-Bilddaten (BD-LDCT) auf Basis der erfassten LDCT-Projektionsmessdaten (PMD-LDCT),
- eine Entscheidungsunterstützungseinrichtung (43) zum Anwenden von Verfahrensschritten zum Ermitteln von Ergebnisdaten (ED-LDCT), welche mit Hilfe eines Verfahrens nach einem der Ansprüche 1 bis 8 an die Verarbeitung von LDCT-Bilddaten angepasst wurden, auf die rekonstruierten LDCT-Bilddaten (BD-LDCT),
- eine Ausgabeeinheit (44) zum Ausgeben einer Information (Inf) zur Unterstützung einer Mehrzahl von Diagnoseentscheidungen auf Basis der ermittelten Ergebnisdaten (ED-LDCT).

13. Computerprogrammprodukt mit einem Computerprogramm, welches direkt in eine Speichereinheit einer Rechnereinheit ladbar ist, mit Programmabschnitten, um alle Schritte eines Verfahrens nach einem der Ansprüche 1 bis 10 auszuführen, wenn das Computerprogramm in der Rechnereinheit ausgeführt wird.

14. Computerlesbares Medium, auf welchem von einer Rechnereinheit ausführbare Programmabschnitte gespeichert sind, um alle Schritte des Verfahrens nach einem der Ansprüche 1 bis 10 auszuführen, wenn die Programmabschnitte von der Rechnereinheit ausgeführt werden.

## Claims

1. Method for adapting method steps for finding a result in a CT-based decision support method to the evaluation of LDCT image datasets (BD-LDCTₙ), having the steps:
- Acquiring a plurality of reference image datasets (R-BDₙ) from a plurality of patients, wherein a reference image dataset (R-BDₙ) in each case at least has:
- A CT image dataset (R-BD-CTₙ) from one (Pₙ) of the plurality of patients,
- An LDCT image dataset (R-BD-LDCTₙ) from the patient (Pₙ) ,
- Applying method steps (PE-VS₀, ED-VS₀) for establishing result data (ED-CT, ED-LDCT) to the different image datasets (R-BD-CTₙ, R-BD-LDCTₙ) of the reference image datasets (R-BDₙ) ,
- Comparing the result data (R-ED-CTₙ, R-ED-LDCTₙ) with one another,
- Adapting the method steps (PE-VS₀, ED-VS₀) for establishing result data (ED-CT, ED-LDCT) to the establishing of result data (ED-LDCT) with reference to an LDCT image dataset (BD-LDCTₙ, R-BD-LDCTₙ) on the basis of a result (VE) of the comparison,
wherein an adaptation to a plurality of different medical problems takes place.

2. Method according to claim 1, wherein the method steps (PE-VS₀, ED-VS₀) for establishing result data (R-ED-CTₙ, R-ED-LDCTₙ) comprise the following steps:
- Applying a method step (PE-VS₀) for parameter extraction to the different image datasets (R-BD-CTₙ, R-BD-LDCTₙ) of the reference image datasets (R-BDₙ), wherein parameter datasets (EP-CTₙ, EP-LDCTₙ) are obtained in each case,
- Applying a method step (ED-VS₀) for establishing result data (R-ED-CTₙ, RE-LDCTₙ) on the basis of the extracted parameter datasets (EP-CTₙ, EP-LDCTₙ) .

3. Method according to claim 2, wherein the adaptation of the method steps (EP-VS₀, ED-VS₀) for establishing result data (ED-CT, ED-LDCT) to the establishing of result data (ED-LDCT) with reference to an LDCT image dataset (BD-LDCTₙ, R-BD-LDCTₙ) comprises
- The adaptation of the method step (EP-VS₀) for parameter extraction to the parameter extraction from an LDCT image dataset (R-BD-LDCTₙ) and/or
- The adaptation of the method step (ED-VS₀) for establishing result data (ED-CT, ED-LDCT) to the establishing of result data (ED-LDCTₙ) with reference to an LDCT image dataset (R-BD-LDCTₙ) .

4. Method according to one of the preceding claims, wherein the result data (ED-CT, ED-LDCT) represents a decision aid in the diagnosis of at least one of the following diseases:
- Pulmonary emphysema,
- Respiratory diseases,
- Interstitial lung diseases,
- Cardiovascular disease,
- Osteoporosis,
- Cancers,
- Bone damage.

5. Method according to one of claims 2 to 4, wherein the adaptation of the method step for parameter extraction is additionally based on a comparison of the extracted parameters (EP-CTₙ, EP-LDCTₙ), which were obtained on the basis of the different image datasets (R-BD-CTₙ, R-BD-LDCTn).

6. Method according to one of claims 2 to 5, wherein the extracted parameter datasets (EP-CTₙ, EP-LDCTₙ) comprise one of the following types of parameter:
- The lung tissue density,
- Bronchial wall analysis data,
- Tissue patterns of the lungs to be recognized,
- The calcium score,
- The myocardial fat,
- The size of the myocardium,
- The bone density,
- Texture features
- Bone structure data.

7. Method according to one of the preceding claims, wherein the adaptation of the method steps (EP-VS₀, ED-VS₀) for establishing result data (ED-CT, ED-LDCT) to the establishing of result data (ED-LDCT) is done with reference to an LDCT image dataset (BD-LDCTₙ, R-BD-LDCTₙ) with the aid of a machine learning method.

8. Method according to one of the preceding claims, wherein for the case in which, after an adaptation of the method steps (EP-VS₀, ED-VS₀) for establishing result data (ED-CT, ED-LDCT) to the establishing of result data (ED-LDCT) with reference to an LDCT image dataset (BD-LDCTₙ, R-BD-LDCTₙ), partly different result data (R-ED-CTₙ, R-ED-LDCTₙ) is created on the basis of the CT image data and the LDCT image data, a value for a probability for a correct establishment of result data (R-ED-LDCT) is established on the basis of LDCT image data (BD-LDCT).

9. LDCT-based decision support method, having the following steps:
- Acquisition of LDCT projection measurement data (LDCT-PMD) from a patient,
- Reconstruction of LDCT image data (LDCT-BD) on the basis of the acquired LDCT projection measurement data (LDCT-PMD),
- Application of method steps for establishing result data (ED-LDCT), which have been adapted with the aid of a method as claimed in one of claims 1 to 8 to the processing of LDCT image data (LDCT-BD), to the reconstructed LDCT image data (LDCT-BD)
- Output of information for supporting a diagnosis decision on the basis of the established result data (ED-LDCT).

10. Method according to claim 9, wherein protocol parameters for the acquisition of LDCT projection measurement data (LDCT-PMD) are adapted to at least one disease to be diagnosed.

11. Adaptation device (30), having:
- An input interface (31) for acquiring a plurality of reference image datasets (R-BDₙ) from a plurality of patients, wherein a reference image dataset (R-BDₙ) in each case has at least:
- A CT image dataset (R-BD-CTₙ) from one (Pₙ) of the plurality of patients,
- An LDCT image dataset (R-BD-LDCTₙ) from the patient (Pₙ),
- A result data establishment unit (32) for applying method steps (EP-VS₀, ED-VS₀) for establishing result data (R-ED-CTₙ, R-ED-LDCTₙ) to the different image datasets (R-BD-CTₙ, R-BD-LDCTₙ) of the reference image datasets (R-BDₙ),
- A comparison unit (33) for comparing the result data (R-ED-CTₙ, R-ED-LDCTₙ) with one another,
- An adaptation unit (34) for adaptation of the method steps (EP-VS₀, ED-VS₀) for establishing result data (R-ED-CTₙ, R-ED-LDCTₙ) to the establishing of result data (ED-LDCT) with reference to an LDCT image dataset (BD-LDCT, BD-LDCT) on the basis of a result (VE) of the comparison, wherein an adaptation to a plurality of different medical problems takes place.

12. System (40) for LDCT-based decision support, having:
- An LDCT imaging device (41) for acquiring LDCT projection measurement data (PMD-LDCT) from a patient and for reconstruction of LDCT image data (BD-LDCT) on the basis of the acquired LDCT projection measurement data (PMD-LDCT),
- A decision support device (43) for applying method steps for establishing result data (ED-LDCT), which has been adapted with the aid of a method as claimed in one of claims 1 to 8 to the processing of LDCT image data, to the reconstructed LDCT image data (BD-LDCT),
- An output unit (44) for output of information (Inf) for supporting a plurality of diagnosis decisions on the basis of the established result data (ED-LDCT).

13. Computer program product with a computer program, which is able to be loaded directly into a memory unit of a computer unit, with program sections in order to carry out all steps of a method according to one of claims 1 to 10 when the computer program is executed in the computer unit.

14. Computer-readable medium, on which program sections able to be executed by a computer unit are stored, in order to carry out all steps of the method according to one of claims 1 to 10 when the program sections are executed by the computer unit.

## Revendications

1. Procédé d'adaptation de stades de procédé pour trouver un résultat dans un procédé d'aide à la décision reposant sur CT à l'exploitation d'ensembles (BD-LDCTₙ) de données d'image LDCT, comportant les stades :
- on acquiert une pluralité d'ensembles (R-BDₙ) de données d'image de référence d'une pluralité de patients, un ensemble (R-BDₙ) de données d'image de référence ayant respectivement au moins :
- un ensemble (R-BD-CTₙ) de données d'image CT de l'un (Pₙ) de la pluralité de patients,
- un ensemble (R-BD-LDCTₙ) de données d'image LDCT du patient (Pₙ),
- on applique des stades (PE-VS₀, ED-VS₀) à la détermination de données (ED-CT, ED-LDCT) de résultat aux ensembles (R-BD-CTₙ, R-BD-LDCTₙ) de données d'image différentes des ensembles (R-BDₙ) de données d'image de référence,
- on compare les données (R-ED-CTₙ, R-ED-LDCTₙ) de résultat entre eux,
- on adapte les stades (PE-VS₀, ED-VS₀) de procédé pour la détermination de données (ED-CT, ED-LDCT) de résultat à la détermination de données (ED-LDCT) de résultat à l'aide d'un ensemble (BD-LDCTₙ, R-BD-LDCTₙ) de données d'image LDCT sur la base d'un résultat (VE) de la comparaison,
dans lequel une adaptation à une pluralité de questions médicales posées a lieu.

2. Procédé suivant la revendication 1, dans lequel les stades (PE-VS₀, ED-VS₀) de procédé pour déterminer des données (R-ED-CTₙ, R-ED-LDCTₙ) de résultat comprennent les stades suivants :
- on applique un stade (PE-VS0) de procédé d'extraction de paramètre aux ensembles (R-BD-CTₙ, R-BD-LDCTₙ) de données d'image différents des ensembles (R-BDₙ) de données d'image de référence, des ensembles (EP-CTₙ, EP-LDCTₙ) de données de paramètre étant obtenues respectivement,
- on applique un stade (ED-VS₀) de procédé de détermination de données (R-ED-CTₙ, RE-LDCTₙ) de résultat sur la base des ensembles (EP-CTₙ, EP-LDCTₙ) de données de paramètre extraits.

3. Procédé suivant la revendication 2, dans lequel l'adaptation des stades (EP-VS₀, ED-VS₀) de procédé de détermination de données (ED-CT, ED-LDCT) de résultat à la détermination de données (ED-LDCT) de résultat à l'aide d'un ensemble (BD-LDCTₙ, R-BD-LDCTₙ) de données d'image LDCT comprend :
- l'adaptation du stade (EP-VS₀) de procédé d'extraction de paramètres à l'extraction de paramètres d'un ensemble (R-BD-LDCTₙ) de données d'image LDCT et/ou
- l'adaptation du stade (ED-VS₀) de procédé de détermination de données (ED-CT, ED-LDCT) de résultat à la détermination de données (ED-LDCTₙ) de résultat à l'aide d'un ensemble (R-BD-LDCTₙ) de données d'image LDCT.

4. Procédé suivant l'une des revendications précédentes, dans lequel les données (ED-CT, ED-LDCT) de résultat représentent une aide à la décision dans le diagnostic d'au moins l'une des maladies suivantes :
- l'emphysème pulmonaire,
- les maladies des voies respiratoires,
- la maladie pulmonaire interstitielle,
- la maladie cardiovasculaire,
- l'ostéoporose,
- les maladies cancéreuses,
- les lésions osseuses.

5. Procédé suivant l'une des revendications 2 à 4, dans lequel l'adaptation du stade de procédé d'extraction de paramètres repose en outre sur une comparaison des paramètres (EP-Ctₙ, EP-LDCTₙ) extraits, qui ont été obtenus sur la base des ensembles (R-BD-CTₙ, R-BD-LDCTₙ) de données d'image différents.

6. Procédé suivant l'une des revendications 2 à 5, dans lequel les ensembles (EP-CTₙ, EP-LDCTₙ) de données de paramètres extraits contiennent l'un des types de paramètres suivants :
- la densité du tissu pulmonaire,
- des données d'analyses de la paroi des bronches,
- un modèle de tissu à détecter du poumon,
- un taux de calcium,
- la graisse du myocarde,
- la taille du myocarde,
- la densité osseuse,
- des caractéristiques de texture,
- des données de structure osseuse.

7. Procédé suivant l'une des revendications précédentes, dans lequel on effectue l'adaptation des stades (EP-VS₀, ED-VS₀) de procédé de détermination de données (ED-CT, ED-LDCT) de résultat à la détermination de données (ED-LDCT) de résultat à l'aide d'un ensemble (BD-LDCTₙ, R-BD-LDCTₙ) de données d'image LDCT au moyen d'un procédé d'apprentissage automatique.

8. Procédé suivant l'une des revendications précédentes, dans lequel, dans le cas où, après une adaptation des stades (EP-VS₀, ED-VS₀) de procédé de détermination de données (ED-CT, ED-LDCT) de résultat à la détermination de données (ED-LDCT) de résultat à l'aide d'un ensemble (BD-LDCTₙ, R-BD-LDCTₙ) de données d'image LDCT sur la base des données d'image CT et des données d'image LDCT on produit des données (R-ED-CTₙ, R-ED-LDCTₙ) de résultat différentes, on détermine une valeur de probabilité d'une détermination correcte de données (R-ED-LDCT) de résultat sur la base de données (BD-LDCT) d'image LDCT.

9. Procédé d'aide à la décision reposant sur LDCT comportant les stades :
- on acquiert des données (LDCT-PMD) de mesure de projection LDCT d'un patient,
- on reconstruit des données (LDCT-BD) d'image LDCT sur la base des données (LDCT-PMD) de mesures de projection LDCT-actives,
- on applique des stades de procédé de détermination des données (ED-LDCT) de résultat, qui ont été adaptées à l'aide d'un procédé suivant l'une des revendications 1 à 8, au traitement de données (LDCT-BD) d'image LDCT, aux données (LDCT-BD) d'image LDCT reconstruites,
- on émet une information d'aide à une pluralité de décisions de diagnostic sur la base des données (ED-LDCT) de résultat déterminées.

10. Procédé suivant la revendication 9, dans lequel on adapte des paramètres de programme d'acquisition de données (LDCT-PMD) de mesures de projection LDCT à au moins une maladie à diagnostiquer.

11. Dispositif (30) d'adaptation, comportant :
- une interface (31) d'entrée d'acquisition d'une pluralité d'ensembles (R-BDₙ) de données d'image de référence d'une pluralité de patients, un ensemble (R-BDₙ) de données d'image de référence ayant au moins respectivement :
- un ensemble (R-BD-CTₙ) de données d'image CT de l'un (Pₙ) de la pluralité de patients,
- un ensemble (R-BD-LDCTₙ) de données d'image LDCT du patient (Pₙ),
- une unité (32) de détermination des données de résultat pour l'application de stades (EP-VS₀, ED-VS₀) de procédé de détermination de données (R-ED-CTₙ, R-ED-LDCTₙ) de résultat aux ensembles (R-BD-CTₙ, R-BD-LDCTₙ) de données d'image différents des ensembles (R-BDₙ) de données d'image de référence,
- une unité (33) de comparaison pour comparer les données (R-ED-CTₙ, R-ED-LDCTₙ) de résultat entre elles,
- une unité (34) d'adaptation des stades (EP-VS₀, ED-VS₀) de procédé de détermination de données (R-ED-CTₙ, R-ED-LDCTₙ) de résultat à la détermination de données (ED-LDCT) de résultat à l'aide d'un ensemble (BD-LDCT, BD-LDCT) de données d'image LDCT sur la base d'un résultat (VE) de la comparaison, une adaptation s'effectuant sur une pluralité de questions médicales posées.

12. Système (40) d'aide à la décision reposant sur LDCT, comportant :
- un dispositif (41) d'imagerie LDCT pour acquérir des données (PMD-LDCT) de mesure de projection LDCT d'un patient et pour reconstruire des données (BD-LDCT) d'image LDCT sur la base des données (PMD-LDCT) de mesure de projection LDCT acquises,
- un dispositif (43) d'aide à la décision pour l'application de stades de procédé de détermination de données (ED-LDCT), qui, à l'aide d'un procédé suivant les revendications 1 à 8, ont été adaptés au traitement de données d'image LDCT, aux données (BD-LDCT) d'image LDCT reconstruites,
- une unité (44) d'émission pour émettre une information (Inf) d'aide à une pluralité de décisions de diagnostic sur la base des données (ED-LDCT) de résultat déterminées.

13. Produit de programme d'ordinateur comprenant un programme d'ordinateur, qui peut être chargé directement dans une unité de mémoire d'une unité informatique, comprenant des parties de programme pour effectuer tous les stades d'un procédé suivant l'une des revendications 1 à 10, lorsque le programme d'ordinateur est réalisé dans l'unité informatique.

14. Support déchiffrable par ordinateur, sur lequel des parties de programme pouvant être réalisées par une unité informatique sont mises en mémoire, pour effectuer tous les stades du procédé suivant l'une des revendications 1 à 10, lorsque les parties de programme sont réalisées par l'unité informatique.
